## Europäisches Patentamt
## European Patent Office
## Office européen des brevets

(19)

(11) Veröffentlichungsnummer: **0 101 581**
**B1**

(12) **EUROPÄISCHE PATENTSCHRIFT**

(45) Veröffentlichungstag der Patentschrift:
12.06.85

(51) Int. Cl.⁴: **C 07 C 51/56, C 07 C 53/12**

(21) Anmeldenummer: **83107535.3**

(22) Anmeldetag: **30.07.83**

(54) Verfahren zur Herstellung von Essigsäureanhydrid.

(30) Priorität: **14.08.82 DE 3230307**

(43) Veröffentlichungstag der Anmeldung:
**29.02.84 Patentblatt 84/9**

(45) Bekanntmachung des Hinweises auf die Patenterteilung:
**12.06.85 Patentblatt 85/24**

(84) Benannte Vertragsstaaten:
**BE DE FR GB IT NL**

(56) Entgegenhaltungen:
**DE - A - 1 939 286**
**DE - A - 3 024 353**

(73) Patentinhaber: **HOECHST AKTIENGESELLSCHAFT,
Postfach 80 03 20, D-6230 Frankfurt am Main 80 (DE)**

(72) Erfinder: **Vogt, Wilhelm, Dr., Bellerstrasse 74,
D-5030 Hürth (DE)**
Erfinder: **Jägers, Erhard, Dr., Schwarzwaldstrasse 1,
D-5303 Bornheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

Die DE-A Nr. 2450965 beschreibt bereits die Herstellung von Essigsäureanhydrid aus Essigsäuremethylester (Methylacetat) und Kohlenmonoxid, wobei man die Reaktionspartner bei Drücken von 1 bis 500 bar und Temperaturen von 50 bis 250° C (323 bis 523 K) über Katalysatoren leitet, die Edelmetalle der Nebengruppe VIII des Periodensystems oder deren Verbindungen sowie Jod und/oder Jodverbindungen enthalten. Als Promotoren können Alkyl- oder Arylphosphine oder auch organische Stickstoffverbindungen, wie Pyridine, Pyrrolidone, Alkylamine oder N-Alkyl-Derivate des Anilins fungieren. Als weitere mögliche Promotoren werden carbonylbildende Metalle oder deren Verbindungen, z. B. Cobalt, Nickel oder Eisen, gekannt. Die Anwesenheit grösserer Mengen, z. B. 5 bis 50 Vol.-%, an Wasserstoff im Reaktionsgas wirkt sich in vielen Fällen sehr günstig auf den Reaktionsablauf aus. Der einzusetzende Essigsäuremethylester kann unter den Reaktionsbedingungen auch aus Dimethylether gebildet werden.

Aus der EP-B Nr. 8396 ist es zur Herstellung von Essigsäureanhydrid weiter bekannt, als Promotor mindestens eine heterocyclische aromatische Verbindung, in der mindestens ein Heteroatom ein quaternäres Stickstoffatom ist und deren Schmelz- oder Mischschmelzpunkt unterhalb 413 K, dem Siedenpunkt von Essigsäureanhydrid, liegt, einzusetzen und zusätzlich in Gegenwart einer aliphatischen Carbonsäure mit 1 bis 8 C-Atomen zu arbeiten. Auch hier können Kohlenmonoxid/Wasserstoffgemische mit bis zu 10 Vol.-% Wasserstoff eingesetzt werden. Als Ausgangsstoffe kommen Methylacetat und/oder Dimethylether in Frage.

Weiterhin betrifft auch die DE-C Nr. 2610036 u. a. die Herstellung von Essigsäureanhydrid aus Methylacetat oder Dimethylether, CO, einen Jodid oder Bromid, unter praktisch wasserfreien Bedingungen in Gegenwart eines Edelmetallkatalysators aus der Gruppe VIII des Periodensystems und eines Mehrfachpromotors, der ein Metall der Gruppen IVA, Va oder VIA des Periodensystems sowie eine Organostickstoffverbindung oder Organosphosphorverbindung enthält, worin Stickstoff und Phosphor dreibindig sind. Wasserstoff soll jedoch, wenn überhaupt, nur in Spuren im Verfahren gegenwärtig sein (Spalte 5, Zeilen 46-51). Die Beispiel 20 und 21 verwenden neben Rhodiumtrichlorid Triphenylphosphin und Vanadiumacetylacetonat bzw. metallisches Niob als Promotoren. Auch alle anderen der insgesamt 24 Beispiel führen nur Rhodiumtrichlorid als Edelmetallkatalysator an.

Es ist also einerseits aus der DE-A Nr. 2450965 bekannt, ggf. in Anwesenheit grösserer Mengen Wasserstoff, jedoch ohne Vanadium oder Niob zu arbeiten, andererseits beschreibt die DE-C Nr. 2610036 die Arbeitsweise in Abwesenheit von Wasserstoff, jedoch in Gegenwart von Vanadium oder Niob.

Schliesslich beschreibt auch die DE-A Nr. 3024353 ein Verfahren zur Herstellung von Essigsäureanhydrid durch Carbonylierung von Methylacetat oder Dimethylether in Gegenwart eines Katalysatorsystems, das als Edelmetall der Gruppe VIII des Periodensystems der Elemente jedoch ausschliesschlich Rhodium- oder Iridiumverbindungen enthält. Neben einem Bromid oder Jodid und einer Organostickstoff- oder Organophosphorverbindung kann das Katalysatorsystem Promotormetalle u. a. aus den Gruppen IVB, VB und VIB (Seite 10, Zeile 23) enthalten, wobei für die Gruppen IVB und VIB Titan bzw. Chrom (Seite 9, Zeile 9) stellvertretend angeführt sind, während die Gruppe VB nicht weiter erläutert wird. Das Kohlenmonoxid kann auch mit Wasserstoff gemischt sein (Seite 8, Absatz 2).

Überraschenderweise wurde nun gefunden, dass eine Arbeitsweise in Gegenwart wesentlicher Mengen Wasserstoff und unter Verwendung von Vanadium oder Niob eine erhebliche Steigerung der Katalysatorleistung bewirkt, wenn als Edelmetall Palladium oder eine seiner Verbindungen eingesetzt wird. Dies ist um so erstaunlicher, als man zufolge dem nachfolgenden Vergleichsbeispiel 1 bei einer Arbeitsweise analog DE-A Nr 2450965 mit einem Palladiumkatalysator ohne Vanadiumpromotor, aber in Gegenwart von Wasserstoff, nur rund 113 g Essigsäureanhydrid/g Pd.· h erhält. Das erfindungsgemässe Beispiel 2 zeigt demgegenüber den überraschenden Effekt, dass die Katalysatorleistung auf 853 g Essigsäureanhydrid/g Pd.· h ansteigt. Die Selektivität der Bildung von Essigsäureanhydrid beträgt dabei rund 90%.

Im einzelnen betrifft die Erfindung nunmehr ein Verfahren zur Herstellung von Essigsäureanhydrid durch Umsetzung von Methylacetat und/oder Dimethylether mit einem aus Kohlenmonoxid und 5 bis 30 Vol.-% Wasserstoff bestehenden Reaktionsgas unter praktisch wasserfreien Bedingungen bei Temperaturen von 390 bis 540 K und Drücken von 1 bis 300 bar in Gegenwart eines Katalysatorsystems aus Edelmetallen der Gruppe VIII des Periodensystems der Elemente oder deren Verbindungen, Jod und/oder dessen Verbindungen sowie einer tertiären oder quaternären organischen Stickstoff-, Phosphor-, Arsen- oder Antimonverbindung, welches dadurch gekennzeichnet ist, dass man mit Palladium oder dessen Verbindungen als Edelmetall in Gegenwart von Vanadium oder Niob oder deren Verbindungen im Katalysatorsystem arbeitet. Vorzugsweise setzt man Methylacetat oder Dimethylether/Palladium(verbindung)/Iod(verbindung)/Stickstoff-, Phosphor-, Arsen- oder Antimonverbindung/Vanadium-, Niob(verbindung) im Molverhältnis 1 : (0,001 - 0,01) : (0,01 - 1) : (0,01 - 1) : (0,0001 - 0,1) ein.

Der Wasserstoff überführt das Reaktionssystem in einen hochaktiven Zustand. Vorzugsweise enthält das Reaktionsgas 5 bis 15 Vol.-% $H_2$ und 95 bis 85 Vol.-% CO.

Als Palladiumverbindungen verwendet man bevorzugt $PdCl_2$ und $Pd(CH_2COO)_2$. Als Jodverbindung wird bevorzugt Methyljodid oder Jodwasserstoff verwendet. Als tertiäre Organostick-

stoff- oder Organophosphorverbindungen kommen Amine, Phosphine oder Aminophosphine in Frage, vorzugsweise Trialkylamine, N,N-Dialkylanilin, Pyridin, Trialkyl- oder Triarylphosphine, insbesondere N-Methylimidazol, 3-Picolin, 2,4-Lutidin, 3,4-Lutidin, Chinolin, Tributylphosphin, Trioctylphosphin, Trilaurylphosphin oder Triphenylphosphin. Die Organostickstoff- oder Organophosphorverbindungen können jedoch auch quaternisiert mit Methylhalogenid oder Halogenwasserstoff eingesetzt werden, z. B. als N-Methylpyridiniumhalogenid, N,N-Dimethylimidazoliumhalogenid, N-Methyl-3-picoliniumhalogenid, N-Methyl-2,3-lutidiniumhalogenid, N-Methyl-3,4-Lutidiniumhalogenid, N-Methylchinoliniumhalogenid, Tributylmethylphosphoniumhalogenid, Trioctylmethylphosphoniumhalogenid, Trilaurylmethylphophoniumhalogenid, Triphenylmethylphosphoniumhalogenid, wobei unter Halogenid jeweils Chlorid, Bromid oder vorzugsweise Jodid zu verstehen sind. Als organische Arsen- und Antimonverbindungen können Arsine und Stibine mit Vorteil verwendet werden.

Als Vanadium- und Niobverbindungen können z. B. die Chloride ($VCl_3$, $NbCl_5$) oder Acetylacetonate ($V(C_6H_7O_2)_3$) eingesetzt werden. Palladium und Vanadium bzw. Niob werden bevorzugt im Molverhältnis (1:0,5) bis (1:20) eingesetzt.

Bevorzugt wird das Verfahren der Erfindung bei Temperaturen von 400 bis 475 K und Drücken von 20 bis 150 bar durchgeführt.

Die erfindungsgemässe Carbonylierungsreaktion kann auch in Gegenwart von Lösemitteln durchgeführt werden. Als solche sind Essigsäure oder Amide wie N-Methylpyrrolidon, N,N-Dimethylacetamid oder schwefelhaltige Lösemittel wie Sulfolan besonders geeignet.

Besonders im Falle des Systems Pd/V ist der gefundene Effekt von besonderer wirtschaftlicher Bedeutung, weil die geologischen Vorkommen des Palladiums etwa 15 mal so gross wie die des Rhodiums sind. Somit führt das durch die Kombination der beschriebenen Massnahmen gekennzeichnete Verfahren der Erfindung zu einer wesentlichen Reduzierung der Katalysatorkosten.

Trotz der Gegenwart von Wasserstoff liegt die auf eingesetztes CO bezogene Selektivität der Methanbildung nicht über 1%, während eine Selektivität an Essigsäureanhydrid, bezogen auf das umgesetzte Methylacetat bzw. Dimethylether, von 90% erreichbar ist.

*Beispiel 1* (Vergleichsbeispiel)

250 g (3,38 mol) Methylacetat, 50 g (0,352 mol) $CH_3J$, 1,06 g (6 mmol) $PdCl_2$ und 102 g (0,296 mol) Methyltributylphosphoniumjodid werden in einem Hastelloy-Autoklaven mit 100 bar $CO/H_2$ im Molverhältnis 10:1 bei 455 K umgesetzt. Nach 1,5 h werden 108 g (1,06 mol) Essigsäureanhydrid gefunden, entsprechend 113,2 g $Ac_2O/g$ Pd·h. 106,2 g (1,44 mol) Methylacetat werden nicht umgesetzt zurückerhalten. Die Selektivität beträgt 54,7%.

*Beispiel 2*

250 g (3,38 mol) Methylacetat, 1,06 g (6 mmol) $PdCl_2$, 1,39 g (9 mmol) $VCl_3$, 50 g (0,352 mol) Methyljodid und 102 g (0,296 mol) Methyltributylphosphoniumjodid werden bei 455 K mit 100 bar $CO/H_2$ im Molverhältnis 10:1 umgesetzt. Nach 19 min Reaktionszeit findet man 171,8 g (1,68 mol) Essigsäureanhydrid entsprechend 853 g $Ac_2O/g$ Pd·h neben Spuren von Ethylidendiacetat. 111,5 g (1,51 mol) Methylacetat werden nicht umgesetzt zurückerhalten. Die Selektivität beträgt 89,5%.

*Beispiel 3*

250 g Methylacetat, 1,06 g $PdCl_2$, 3,2 g (12 mmol) Niob-V-chlorid, 50 g Methyljodid und 102 g Methyltributylphosphoniumjodid werden bei 455 K mit 100 bar $CO/H_2$ im Molverhältnis 10:1 umgesetzt. Nach 45 min findet man 139 g Essigsäureanhydrid entsprechend 291,4 g $Ac_2O/g$ Pd·h.

**Patentansprüche**

1. Verfahren zur Herstellung von Essigsäureanhydrid durch Umsetzung von Methylacetat und/oder Dimethylether mit einem aus Kohlenmonoxid und 5 bis 30 Vol.-% Wasserstoff bestehenden Reaktionsgas unter praktisch wasserfreien Bedingungen bei Temperaturen von 390 bis 540 K und Drücken von 1 bis 300 bar in Gegenwart eines Katalysatorsystems aus Edelmetallen der Gruppe VIII des Periodensystems der Elemente oder deren Verbindungen, Jod und/oder dessen Verbindungen sowie einer tertiären oder quaternären organischen Stickstoff-, Phosphor-, Arsen- oder Antimonverbindung, dadurch gekennzeichnet, dass man mit Palladium oder dessen Verbindungen als Edelmetall in Gegenwart von Vanadium oder Niob oder deren Verbindungen im Katalysatorsystem arbeitet.

2. Verfahren nach Anspruch 1, dadurch gekennzeichnet, dass man Methylacetat oder Dimethylether/Palladium(verbindung)/Jod(verbindung)Stickstoff-, Phosphor-, Arsen- oder Antimonverbindung/Vanadium-, Niob(verbindung) im Molverhältnis 1:(0,0001 - 0,01):(0,01 - 1):(0,01 - 1):(0,0001 - 0,1) einsetzt.

**Claims**

1. Process for making acetic anhydride by reacting methyl acetate and/or dimethylether with a reaction gas consisting of carbon monoxide and 5 to 30 vol.-% hydrogen under practically anhydrous conditions at temperatures of 390 to 540 K, under pressures of 1 to 300 bar in the presence of a catalyst system comprised of noble metals belonging to group VIII of the Periodic System of the elements or their compounds, iodine and/or its compounds and a tertiary or quaternary organic nitrogen, phosphorus, arsenic or antimony compound, wherein a catalyst system containing palladium or its compounds as noble metal in the presence of vanadium or niobium or their compounds is used.

2. Process as claimed in Claim 1, wherein the methyl acetate or dimethylether/palladium(compound)/iodine(compound)/nitrogen, phosphorus, arsenic or antimony compound/vanadium or niobium(compound) are used in a molar ratio of 1:(0.0001 - 0.01):(0.01 - 1):(0.01 - 1):(0.0001 - 0.1).

## Revendications

1. Procédé de préparation de l'anhydride acétique par réaction de l'acétate de méthyle et/ou de l'éther diméthylique avec un gaz réactionnel constitué par le monoxyde de carbone et 5 à 30% en volume d'hydrogène dans des conditions pratiquement anhydres à des températures de 390 à 540 K et sous des pressions de 1 à 300 bar en présence d'un système catalytique de métaux nobles appartenant au groupe VIII de la classification périodique des éléments ou de leurs composés, d'iode et/ou de ses composés ainsi que d'un composé d'azote, de phosphore, d'arsenic ou d'antimoine organique tertiaire ou quaternaire, caractérisé en ce que l'on utilise un système catalytique contenant du palladium et/ou ses composés comme métal noble en présence de vanadium ou de niobium ou de leurs composés.

2. Procédé selon la revendication 1, caractérisé en ce que l'on utilise l'acétate de méthyle ou l'éther diméthylique/le (composé de) palladium/ le (composé de) iode/ le composé d'azote, de phosphore, d'arsenic ou d'antimoine/le (composé de) vanadium ou de niobe dans un rapport molaire de 1:(0,0001 - 0,01):(0,01 - 1):(0,01 - 1):(0,001 - 0,1).